# EUROPEAN PATENT SPECIFICATION

(11) **EP 4 346 847 B1**
(45) Date of publication and mention of the grant of the patent: **17.09.2025**
(21) Application number: 22732150.2
(22) Date of filing: 03.06.2022
(51) Int. Cl.: A61K 33/06, A61K 33/14, A61K 9/06, A61K 9/00, A61P 29/00, A61K 9/70

(54) **PLURI-IONIC COMPLEX FOR USE IN THE PREVENTION OR TREATMENT OF NEUROGENIC INFLAMMATION**
PLURI-IONISCHEKOMPLEXE ZUR VERWENDUNG BEI DER VORBEUGUNG ODER BEHANDLUNG VON NEUROGENER ENTZÜNDUNG
COMPLEXE PLURI-IONIQUE POUR SON UTILISATION DANS LA PRÉVENTION OU LE TRAITEMENT DE L'INFLAMMATION NEUROGÈNE

(30) Priority: 04.06.2021 BE 202105448
(43) Date of publication of application: 10.04.2024
(73) Proprietor: Olys Pharma Srl, 4537 Verlaine (BE)
(72) Inventor: VAN WESEPOEL, Philippe, 13008 Marseille (FR); PLUCKER, Jean-François, 1180 Uccle (BE)
(74) Representative: Kirkpatrick
(86) International application number: PCT/EP2022/065271
(87) International publication number: WO 2022/254042

(56) References cited:
- WO-A1-2009/006701
- WO-A1-2013/081366
- AU-B2- 2008 274 908
- CN-A- 108 310 287
- KR-A- 20200 109 179
- US-A- 3 639 625
- US-A- 5 766 639
- TADAO SHIMIZU ET AL: "Intrathecal lithium reduces neuropathic pain responses in a rat model of peripheral neuropathy", INTERNATIONAL ASSOCIATION FOR THE STUDY OF PAIN, ELSEVIER, vol. 85, 1 January 2000 (2000-01-01), pages 59 - 64, XP007920325, DOI: 10.1016/S0304-3959(99)00249-3
- GISÈLE PICKERING ET AL: "Oral magnesium treatment in patients with neuropathic pain: a randomized clinical trial", MAGNESIUM RESEARCH, 1 June 2011 (2011-06-01), England, pages 28, XP055454869, Retrieved from the Internet <URL:http://www.jle.com/download/mrh-289068-oral_magnesium_treatment_in_patients_with_neuropathic_pain_a_randomized_clinical_trial--WpVsvX8AAQEAADeFdfMAAAAB-a.pdf> DOI: 10.1684/mrh.2011.0282
- RICHARDSON PAUL G. ET AL: "Complications of Multiple Myeloma Therapy, Part 1: Risk Reduction and Management of Peripheral Neuropathy and Asthenia", vol. 8, no. Suppl 1, 1 February 2010 (2010-02-01), US, pages S - 4, XP055893118, ISSN: 1540-1405, Retrieved from the Internet <URL:http://dx.doi.org/10.6004/jnccn.2010.0115> DOI: 10.6004/jnccn.2010.0115
- DATABASE GNPD [online] MINTEL; 19 September 2011 (2011-09-19), ANONYMOUS: "Total Hair Removal Kit", XP055892532, retrieved from https://www.gnpd.com/sinatra/recordpage/1612941/ Database accession no. 1612941

## Description

### Field of invention

The invention is in the field of a therapeutic response to prevent and / or treat neurogenic inflammation and restore cellular homeostasis.

### Context

When a cell suffers damage or is subject to stress (of psychic, metabolic, chemical origin...) it induces a depletion of intracellular magnesium Mg²⁺, with concentrations that can drop from 40 to 60% in case of damage *(*Neuropsychiatric Disease and Treatment 2017: 13 275-302*).* However, small fluctuations in the intracellular concentration of Mg²⁺ can influence cellular processes. The dysregulation of Mg²⁺ is frequently observed in patients suffering from diabetes, neurodegenerative diseases as well as metabolic syndrome... *(*Magnesium Is a Key Player in Neuronal Maturation and NeuropathologyInt. J. Mol. Sci. 2019, 20, 3439*).*

Magnesium acts as a cofactor in more than 300 enzymatic reactions where it is crucial for the metabolism of adenosine triphosphate (ATP), an energy source *(*Magnesium in Prevention and Therapy Nutrients 2015, 7*).*

Magnesium has powerful anti-oxidant, anti-necrotic and anti-apoptotic effects. Mg²⁺ is itself largely cytoprotective, cardioprotective and neuroprotective against a wide range of insults *(*Neuropsychiatric Disease and Treatment 2017:13 275-302)*.*

Magnesium, the fourth most common mineral element in the body, also plays an essential role in nerve transmission and neuromuscular conduction. Low magnesium levels are associated with elevated glutaminergic neurotransmission, leading to an environment prone to excitotoxicity, which can lead to oxidative stress and neuronal cell death. This process is involved in several neurological disorders, such as chronic pain *(*The Role of Magnesium in Neurological Disorders, Nutrients, 2018, 10,730*).*

A magnesium deficiency negatively impacts the insulin resistance index (HOMA-IR). Magnesium is also essential for protein synthesis, including DNA and RNA synthesis *(*Magnesium in Prevention and Therapy, Nutrients, 2015, 7). It regulates the transmembrane movement of potassium and calcium.

Based on experiments in which animals are deprived of lithium, lithium is considered an essential nutrient for the functioning of the human body. Unlike other biologically active ions, the concentration of lithium in pluricellular animal fluids is not tightly regulated. Its concentration can vary widely.

Lithium, while having a high biological activity, is tolerated at very variable concentrations of body fluid. The lack of biological regulation of lithium appears to be due to the absence of lithium-specific binding sites and selectivity filters. Lithium therefore exerts its numerous physiological and biochemical effects by competing with other elements for relatively specific macromolecular sites of other cations, particularly sodium and magnesium *(*Towards a unified understanding of Lithium action, Topical Review, 2017, 586).

Lithium and magnesium possess similar ion radii (0.60 and 0.65 Angström, respectively) and similar physico-chemical properties allowing them to successfully compete to bind to several sites of dependent magnesium enzymes *(*Lithium: the pharmacodynamic actions of the amazing ion Ther. Adv. Psychopharmacol. (2013) 3(3) 163-176*).* These ionic radii allow lithium and magnesium to easily cross cell membranes.

The Li⁺ ion activates survival and recovery mechanisms such as inhibition of inositol monophosphatase (IMPase) / inositol polyphosphate 1-phosphatase (IPPase, glycogen synthase kinase 3 (GSK-3)). Thus, lithium produces remarkable protective, anti-apoptotic, anti-anoxic, cellular plasticity and resilience responses. *(*A fully integrated new paradigm for lithium's mode of action - lithium utilizes latent cellular fail-safe mechanisms - Neuropsychiatric Disease and Treatment 2017:13 275-302)*.*

Lithium's target, glycogen synthase kinase 3 (GSK-3), is a serine/threonine kinase, which plays a regulatory role in cellular metabolism in mammals. It regulates neurogenesis, neuronal polarization and axon growth in the developing central nervous system. It is constitutively active in all tissues *(*Role of glycogen synthase kinase-3b in ketamine-induced developmental neuroapoptosis in rats - British Journal of Anaesthesia 110 (S1): i3-i9 (2013*)).*

Lithium inhibits GSK-3, thus improving the activity of BDNF (brain-derived neurotrophic factor) as shown in vitro and in vivo. The role of lithium in increasing BDNF expression plus the role of BDNF in neuron survival has led to the suggestion that lithium plays a role in the treatment of neurodegenerative diseases. The Wnt signaling pathway is involved in neurodegenerative diseases and cancer. Lithium inhibition of GSK-3 has been shown to specifically inhibit the Wnt signaling pathway *(*Towards a unified understanding of Lithium action, Topical Review, 2017, 586).

Preventive lithium treatment, through inhibition of GSK3β, prevents increased taxol-induced GSK3β activity in rats, which simultaneously reduces AKT (protein kinase B) and mTOR (mechanistic target of rapamycin) activities, thereby preventing the development of taxol-induced neuropathic pain ( Inhibition of glycogen synthase kinase 3beta activity with lithium prevents and attenuates paclitaxel-induced neuropathic pain Neuroscience. 2013 December 19; 254*).*

Lithium has anti-inflammatory properties that reduce both pro-inflammatory cytokines and interleukin TNF-alpha levels. On the other hand, lithium regulates the biosynthesis of different neurotransmitters and / or associated receptors such as serotonin and glutamate. Lithium has an anti-allodynia effect as well as a stimulating effect on the production of cerebral beta-endorphin, a MOR agonist with strong analgesic properties. *(*Lithium reverses mechanical allodynia through a µ opioid-dependent mechanism Molecular Pain 2018 Volume 14: 1-8*)*

Lithium also contributes to calcium homeostasis and blocks calcium activation of dependent-pro-apoptotic signaling pathways that confirms its cyto-protective action *(Molecular actions and therapeutic potential of lithium in preclinical and clinical studies of CNS disorders* Pharmacol Ther. 2010 November; 128(2): 281-304*).*

Because the Li⁺ ion inhibits the GSK-3 enzyme which is an important enzyme at the crossroads of several signaling systems, it impacts pluriple downstream targets, including: ionotropic glutamate signaling, pluriple transcription factors, and the Wingless (Wnt)/β catenin-related integration pathway. Wnt signaling plays a role in structural brain processes such as neuronal development, synapse formation, and neuronal plasticity. *(*LITHIUM IN THE TREATMENT OF BIPOLAR DISORDER: PHARMACOLOGY AND PHARMACOGENETICS Mol Psychiatry. 2015 June; 20(6): 661-670*).*

The two-metal-phosphate ATP-Mg-Li complex forms at normal concentrations of ATP and Mg2+ found in plasma or cytoplasm. In addition, high ATP levels corresponding to biological activity or in response to stress in the cytoplasm, organelles (e.g. mitochondria), and extracellular matrix serve as potential reservoirs for accumulating lithium (Li⁺) ions. The two-metal-phosphate complex ATP-Mg-Li is found to be the bioactive form of lithium through co-binding with Mg2+ to phosphates having ligands or receptor cofactors or enzymes. The ATP-Mg-Li complex makes it possible that lithium ions can act by modulating the normal function of ATP as a ligand of cell surface purine receptors, of which there are two subtypes: P2X, which are ion channels that mediate the influx of extracellular calcium ions (Ca2⁺) into the cytoplasm, and P2Y, which are G protein-coupled receptors (GPCRs) that activate the second messenger pathway of inositol trisphosphate to release calcium ions from intracellular reserves, to modulate signaling in the central nervous system and periphery. *(*A Molecular Model for Lithium's Bioactive Form - Biophysical Journal 111, 294-300, July 26, 2016*) .*

Potassium is also an essential element. It is the most abundant cation in intracellular fluid, where it plays a key role in maintaining cellular function, especially in excitable cells such as muscles and nerves. Potassium deficiency is associated with glucose intolerance and diabetes. The interaction of K+ with extracellular Na+ increases the water movement promoting ionic exchanges and balances, including those of lithium *(*Potassium Intake, Bioavailability, Hypertension, and Glucose Control Nutrients 2016, 8, 444*).*

Deregulation these three ions activity is involved in neurogenic inflammation, particularly in the Neuro-Immuno-Cutaneous-Endocrinien (NICE) system. Neurogenic inflammation is associated with neuropathic pain (diabetic peripheral neuropathic pain, neuropathic pain following medical oncological treatment,... ), musculoskeletal pain (lower back pain, fibromyalgia, osteoarthritis, complex regional pain syndrome), orofacial pain, post-herpetic pain, rosacea, psoriasis, atopic dermatitis, prurigo noduralis, urticaria, painful or non-painful scars (burn scars, surgical, ...), diabetic foot (dryness - calluses - ulcers, ... ) and wounds among others.

These deregulations are impactful individually and in conjunction.

A simultaneous balanced approach is justified in order to provide a therapeutic response to neurogenic inflammation and its physiological repercussions such as cell degeneration, hyperkeratosis, xerosis, flaking, wound, and, burning sensations, electric shocks, tingling, itching as well as peripheral pain at the neurocutaneous level, among others...

### Solution of the invention

It has been discovered by the present inventors that a composition containing a particular mixture of lithium, magnesium and potassium, in specific molar ratios, allows, and it alone, to prevent and / or treat neurogenic inflammation in a particularly effective way, especially in topical administration.

The invention relates to a bioactive synergistic pluri-ionic complex providing potentiated biological responses to neurogenic inflammation.

It is a pluri-ionic composition comprising mineral salts, including at least one mineral salt of lithium, of magnesium and of potassium, in the following molar ratios:
lithium 1 - magnesium [0.13 - 0.34] - potassium [1.20-2.40],

Preferably, the lithium concentration in the composition being between 0.1 and 10 mmol/kg.

Preferably, the molar ratio of magnesium to lithium is [0.14 - 0.32], preferably [0.15 - 0.30], preferably [0.18 - 0.25]. Preferably, the molar ratio of potassium to lithium is [1.30 - 2.30], preferably [1.30 - 2.15], preferably [1. - 2.15], preferably [1.55-1.75], preferably [1.58-1.72], preferably again [1.61-1.70].

A mineral salt according to the invention refers to a salt comprising no carbon, with the exception of carbonate salts.

This bioactive pluri-ionic complex consists of a novel combination of "magnesium - lithium - potassium" according to a specific algorithm of molar ratios. The adaptation of one of the components, depending on the therapeutic response to neurogenic inflammation, leads to the proportional adaptation of the concentrations of the other two elements.

This novel algorithmic combination allows, in particular by topical application, simultaneously to break the pro-inflammatory vicious cycle that feeds itself, to restore cellular homeostasis in these ions that is found altered in many pathologies, to promote dynamic competition for the binding sites of these ions in order to obtain corrective physiological responses (enzymatic, metabolic, neurotransmissions...), and to generate a potentiated synergistic response that will strengthen the cellular response to the insult in order to manage neurogenic inflammation quickly and sustainably.

Its action promotes the restoration of homeostasis altered by neurogenic inflammation, in particular that of magnesium and its ionic form Mg²⁺, whose concentrations can be significantly impacted, and contributes to restoring optimal cellular physiological functioning, improving cellular metabolism and restoring cyto-protective functions at the skin and neurocutaneous levels, as well as anti-apoptosis activity at the neuronal level.

Its action promotes potassium rebalancing and restores its key role in maintaining cellular function, especially in excitable cells such as nerve cells. In addition, it promotes the interaction of potassium with sodium, in ionic form K⁺ with extracellular Na⁺ and increases fluid movements promoting ionic exchanges and balances, especially those of lithium. Lithium has a strong biological activity, as it does not have its own system responsible for regulating its concentration which can vary significantly in intra- and extra-cellular fluids, its external intake must be adapted according to the therapeutic need, coordinated and weighted.

The bioactive pluri-ionic complex brings together lithium, magnesium and potassium, which exert their multiple physiological and biochemical effects, described above.

Lithium combines its important anti-inflammatory properties with regulatory actions of neurotransmitters biosynthesis such as serotonin and glutamate, a stimulating effect on the production of cerebral beta-endorphin reducing pain.

Lithium and magnesium are linked. The ATP-Mg-Li two-metals-phosphate complex is found to be the bioactive form of lithium acting by co-binding with Mg²⁺ to phosphates having ligands or receptor cofactors or enzymes. This two-metal-phosphate complex forms at normal concentrations of ATP and Mg²⁺ found in plasma or cytoplasm. In addition, in stressful situations that will raise ATP levels in the cytoplasm, organelles such as mitochondria, the extracellular matrix will serve as potential reservoirs to accumulate Li⁺.

Only the specific combination of molar ratios according to the invention ensures an absence of cellular toxicity, thus guaranteeing compliance with the homeostasis of tissues, especially that of more sensitive nervous tissues, and makes it possible to obtain an important efficacy for the prevention and/or treatment of neurogenic inflammation. Only the compositions meeting the definition according to the invention allow in particular a protective action of neurons, a protective and restorative action of neurites (axons-dendrites) and myelin sheaths against damage caused by cisplatin, treatment recognized as an inducer of neurogenic inflammation. Without prejudging the mechanisms underlying the achievement of such a surprising effect, it can be thought that only this combination of lithium, magnesium and potassium, in the molar ratios according to the invention, makes it possible to optimize the biological activities of each of these elements, in the complex environment of the human and animal organism, involving both inhibitory effects at the level of the pro-inflammatory vicious cycle, a restoration of cellular homeostasis in these ions that are found altered, in particular that of magnesium, and its ion form Mg2 + essential for the optimal functioning of mitochondrias, a dynamic competition effect at the level of ion channels as well as for the sites of fixation of these ions, associated with an increase in intra- and extracellular fluid movements in order to reduce the toxicity of lithium, in particular, an activation of immune, enzymatic, metabolic and neurotransmission responses, a potentiated synergistic response that will strengthen resistance and cellular response to insult by a cyto-protective action at the skin and neurocutaneous levels, a restoration of the potential for neuronal excitability, as well as a inhibition of apoptosis pathways activated by extracellular ATP.

Lithium is present in the composition at a concentration between 0.1 and 100 mmol/kg. The concentration of lithium in the composition is preferably between 0.5 and 50 mmol/kg, preferably still between 1 and 25 mmol/kg, for example between 1.1 and 6 mmol/kg, or between 1.7 and 6 mmol/kg. In case of topical application, targeted cells effective exposition depends on the formulation and/or state of the skin. Ideally, targeted cells are not exposed at concentrations higher than 10 mmol/kg otherwise risking that toxicity surpasses benefits for neurogenic inflammation treatment. Below 0.1 mmol/kg, its effectiveness is also insufficient.

In the context of a topical application, the composition of the invention, simultaneously aims to restore cellular homeostasis in these ions, in particular Mg²⁺, to promote dynamic competition for fixation sites, and to generate a potentiated synergistic response.

Preferably, the composition according to the invention is an aqueous composition, preferably having a pH between 6.0 and 7.5. The pH is adjusted, if necessary, with the help of buffer solutions, so that, depending on the vector of topical application, whether it is water in the form of a spray for example, or a gel, cream or gel-cream, or even another vector. This pH will ideally aim for a moderate acidity of the order of 6.5 ± 7.5 in order to optimize cellular and transmembrane ion penetration.

The composition of the invention relates to the prevention and/or treatment of neurogenic inflammation, and in particular at the level of the Neuro-Immuno-Cutaneous-Endocrine system (NICE).

Neurogenic inflammation is associated with neuropathic pain (diabetic peripheral neuropathic pain, neuropathic pain following medical treatment), musculoskeletal pain (lower back pain, fibromyalgia, osteoarthritis, complex regional pain), orofacial pain, post-herpetic pain, rosacea, psoriasis, atopic dermatitis, prurigo noduralis, urticaria, painful or not painful scars (burn scars, surgeries...), diabetic foot (dryness - calluses - ulcers) and wounds among others.

Indeed, this bioactive pluri-ionic complex "magnesium - lithium - potassium" according to the invention provides an adjustable therapeutic response to the physiological and cytological repercussions of neurocutaneous pathologies, such as hyperkeratosis, xerosis, desquamation, wound ..., at the skin level, such as burning sensations, electric shocks, tingling, itching and peripheral pain at the neurocutaneous level, among others...

The composition of the invention may be used for the prevention and / or treatment of skin neurogenic inflammation and the restoration of homeostasis of the Neuro-Immuno-Cutaneous-Endocrine system, in particular topically.

At least one mineral salt of lithium, magnesium and potassium means that these species are present in ionic form in water. The cations Li⁺, Mg²⁺ and K⁺ are associated with one or more anions, that is, they can come from mixed salts and / or each from a different salt.

Advantageously, lithium is introduced into the composition in the form of lithium chloride, lithium hydroxide, lithium carbonate and / or any other pharmaceutically acceptable mineral salt. Preferably, for topical use, the composition includes lithium chloride.

Advantageously, magnesium is introduced into the composition in the form of magnesium chloride, magnesium carbonate, magnesium hydroxide, magnesium oxide, magnesium sulfate, magnesium silicate and / or any other pharmaceutically acceptable mineral salt.

Preferably, for topical use, the composition includes magnesium chloride, optionally in hydrated form.

Advantageously, potassium is introduced into the composition in the form of potassium chloride, potassium bromide, potassium iodide, potassium phosphate, potassium carbonate, potassium hydroxide, potassium silicate, potassium sulfate and / or any other pharmaceutically acceptable mineral salt. Preferably, for topical use, the composition includes potassium chloride.

The composition of the invention may comprise other mineral salts.

The composition may for example comprise at least one silicon salt, preferably a silicate such as sodium silicate (Na₂SiO₃), optionally hydrated, potassium silicate and/or any other pharmaceutically acceptable mineral salt, especially in topical application.

Silicon is a co-factor of prolyl hydroxylase which participates in the stimulation of fibroblasts. It decreases the permeability of capillaries, has an anti-edematous, soothing and refreshing effect, shortens the time of appearance of granulation in deep burns in particular, accelerates epidermization and thus the healing process.

It plays a role in the regulation of the lymphocyte cell cycle that ultimately affects the immune and inflammatory response.

Silicon decreases the level of expression in wounds of endothelial nitric oxide synthase (eNOS), vascular endothelial growth factor (VEGF), fibroblast growth factor (FGF), epidermal growth factor (EGF), activated B cell light chain kappa-relieving nuclear factor (NF-κB), various cytokines (TNF-α and IL-1β).

Depending on the cutaneous neurogenic inflammation underlying the proposed pathology, the proportion of silicon in relation to other salts is adapted.

The composition may for example comprise at least one mineral salt of manganese, such as manganese chloride, manganese sulfate, manganese carbonate, and / or any other mineral salt of manganese pharmaceutically acceptable, especially in topical application.

Manganese is involved in the physiology and biology of nervous tissues via its role as a cofactor in many enzymatic processes. It also has a beneficial effect for wound healing via the modulation of integrins causing the acceleration of keratinocyte migration. Manganese also plays an essential role in regulating glucose tolerance by acting synergistically with magnesium, which allows in particular the reduction of nervous and epidermal metabolic stress.

The composition may for example include at least one silver mineral salt, such as silver nitrate for its antibacterial properties, for the treatment of chronic wounds (ulcers, bedsores), acute wounds (burns, traumatic wounds, surgical wounds etc ...).

The composition may for example comprise at least one mineral salt of zinc, such as citrate, oroate, zinc sulfate ... which has an important role in many vital enzymatic processes such as DNA and protein synthesis, wound healing, insulin metabolism, development and proper functioning of the nervous system...

The composition may for example comprise at least one copper mineral salt, such as copper carbonate. Copper is the constituent of several enzymes, which are involved in the metabolism of carbohydrates, fats and iron. It has an antioxidant role.

A topical composition is a composition intended to be applied directly to the part of the body to be treated (curative or preventive). It can be the skin. It can also be a mucosa, such as the oral mucosa. The composition of the invention may be for skin use, i.e. intended for external use on the skin. Preferably, the composition is in the form of cream or gel, that is, it may contain the excipients necessary for the formation of a gel or cream.

The mineral salts of the composition of the invention are dissolved in water, that is to say that the main solvent of the composition is preferably water. The water then represents at least 50% by weight of the composition, preferably at least 75% by weight, at least 80%, and preferably at least 90%. The composition may include other liquid ingredients, in particular to promote the dermal absorption of mineral salts, such as fatty substances, emollients, emulsifiers, surfactants, glycerin, or any other agent deemed useful by those skilled in the art.

The composition of the invention may also comprise other molecules, additives or excipients, for example preservatives, antibiotics, stabilizing agents, thickeners, antioxidants. These additives may be useful for the preservation of the composition of the invention, as well as for the final texture of the composition.

For example, gelling agents can be used to formulate the composition in the form of a gel to spread on the skin. For a formulation in the form of a spray, the viscosity will preferably remain rather low.

The composition may for example include one or more vitamins, such as vitamin E. Vitamin E is a powerful antioxidant and modulates immune function.

The invention also relates to a method of preparing the composition comprising the steps of:
- If necessary, the pH of an aqueous solution is adjusted between 6.5 and 7.5;
- One or more concentrated solutions comprising at least one mineral salt of lithium, of magnesium and of potassium is added to the aqueous solution to obtain the following molar ratios:
   lithium 1 - magnesium [0.13 - 0.34] - potassium [1.20-2.40].

Preferably, a lithium concentration of between 0.1 and 10 mmol/kg is obtained.

Preferably, one or more concentrated solutions comprising at least one mineral salt of lithium, magnesium and potassium is added at a temperature above 50 ° C.

When the composition contains a silicon salt, it is previously dissolved in an isolated aqueous solution, preferably added prior to pH adjustment. The pH of this solution can be adjusted, using hydrogen chloride for example, optionally buffered by sodium hydroxide for example, to reach values between 6.5 and 7.5 and then added to the composition containing lithium, magnesium and potassium.

A concentrated solution comprising at least one mineral salt of lithium, magnesium and / or potassium is preferably concentrated 100 to 1000 times compared to the final concentration of the composition, preferably 300 to 800 times.

The solubility limit of the salts used determines the maximum potential concentration of this concentrated solution. The use of a solution concentrated in minerals avoids the reprecipitation of these minerals. For this, pH adjustment is essential.

The invention also relates to a topical composition according to the invention, comprising, in water, at least one mineral salt of lithium, magnesium and potassium, characterized by the following molar ratios: lithium 1 - magnesium [0.13 - 0.34] - potassium [1.20 - 2.40], and a lithium concentration between 0.1 and 10 mmol / kg, for dermal treatment.

The topical composition is preferably a solution, gel, cream or spray.

The invention relates to the use of the composition according to the invention as a medicine, for human or veterinary use.

It also relates to the use of the composition according to the invention as a medical device.

It also relates to the non-therapeutic cosmetic use of the composition according to the invention.

Preferably, the composition according to the invention is used topically, for its therapeutic use as for its cosmetic use.

The composition according to the invention may, in particular, be used for the prevention and/or treatment, in particular topically, of neurogenic inflammation, and in particular to break the pro-inflammatory vicious cycle, ensure protection of neurons, protection and repair of neurites (axons-dendrites) and myelin sheaths, restoration of cellular functioning and tissue homeostasis, especially at the level of the Neuro-Immuno-Cutaneous-Endocrine system.

Neurogenic inflammation may be associated with neuropathic pain (diabetic peripheral neuropathic pain, neuropathic pain following medical treatment), musculoskeletal pain (lower back pain, fibromyalgia, osteoarthritis, complex regional pain), orofacial pain, post-herpetic pain, rosacea, psoriasis, atopic dermatitis, prurigo noduralis, urticaria, painful or non-painful scars (burn scars, surgeries...), diabetic foot (dryness - calluses - ulcers) and / or wounds.

Topical treatment, especially dermal treatment, may be the prevention or treatment of Neuro-Immuno-Cutaneous-Endocrine (NICE) pathologies in which neurogenic inflammation intervenes. Dermal treatment may be intended for skin repair or repair of wounds, including burn wounds. In this case, the presence of silicon is desired, because it confers a cooling effect, it reduces capillary permeability and regulates the passage of mediators and pro-inflammatory immune cells, accelerates granulation and structured re-epithelialization.

The topical composition of the invention for skin repair may advantageously be absorbed on a bandage, preferably packaged in a sterile way.

The invention also relates to a method of preventing and / or treating a disease, in particular neurogenic inflammation, in a subject, comprising administering to the subject a therapeutically effective amount of a composition according to the invention, preferably topically, in particular on the skin and / or mucous membranes.

The subject can be any subject in need thereof, in particular a subject suffering, or likely to suffer, from neurogenic inflammation. It is preferably a human or an animal.

The invention also relates to a cosmetic treatment method comprising administering a composition according to the invention to a subject, preferably topically, in particular on the skin and / or mucous membranes. The subject can be a human or an animal.

The invention will be better understood using the following description.

### Example 1 - Solution 1 concentrated in Sodium metasilicate

45 g sodium metasilicate pentahydrate (212 mmol; CAS 10213-79-3, VWR product No. 28092) are dissolved in 500 g of pure water (milliQ) to give solution 1.

### Example 2 - Solution 2 concentrated in lithium, magnesium and potassium

36.7 g lithium chloride (866 mmol; CAS 7447-41-8; VWR Analar NORMAPUR^{®} Reag. Ph. Eur. for analysis), 38.5 g magnesium chloride hexahydrate (189.4 mmol; CAS 7791-18-6, AMRESCO product No. 0288) and 104.84 g of potassium chloride (1406 mmol; CAS 7447-40-7; VWR Potassium chloride GPR RECTAPUR^{®} ) are dissolved in 500 g of pure water (milliQ) to give solution 2.

### Example 3 - Solution 3 concentrated in manganese

550 mg of Manganese (II) chloride tetrahydrate (2.78 mmol; CAS 13446-34-9, VWR NORMAPUR^{®} ACS for analysis) is dissolved in 500 g of pure water (milliQ) to give solution 3.

### Example 4 - Solution 4

In a container, 10.05 kg of vegetable glycerin, 2.01 kg of cetearyl ethylhaxanoate (Massocare^{™} CO), 1.00 kg of Montanov TM L emulsifier and 0.67 kg of Lipocire^{™} A SG (Gattefossé) are introduced and heated at 82 ° C.

### Example 5 - Composition 1

In a 100kg DUMEK tank equipped with stirring, 50,527 kg of osmosis water is introduced. Was added 72.76 g of solution 1 (or 28.3 mmol of element Si). The solution is heated at 80 °C ± 2 °C and stirred. During the temperature rise of the solution, the pH is adjusted by adding concentrated hydrochloric acid (5.9 g) to reach a pH of 7.13.

Is then added 68.71 g of solution 2 (or 87.5 mmol of Li, 19.1 mmol of Mg and 142.0 mmol of K) and 50.52g of solution 3 (or 0.3 mmol of Mn) and homogenized.

Solution 4 is then added at 82 ° C and homogenized.

Is then cooled at 65 ° C ± 2 ° C and added 737 g of sodium polylacrylate (Safit Care T SP) having here a thickener function then homogenized.

The solution was then cooled at 55 ° C, then introduced 670 g of dimethicone (Xiameter PMX 1503 Fluid) and homogenized. Dimethicone is used here for its film-forming effect, reducing dehydration and providing a soothing effect.

Then 804g of Mikrokill^{™} COS (Lonza) as a preservative is introduced, then homogenized.

The solution is then cooled at 27 ° C and 335 g of vitamin E acetate is added and homogenized to obtain 67.00 kg of the final composition characterized by a pH of 6.1 and a density of 1.018 (a volume 65.81L).

The final concentration of mineral elements of composition 1 (gel - cream) is therefore:
- 0.42 mmol/kg Si
- 1.31 mmol/kg Li
- 0.28 mmol/kg Mg
- 2.12 mmol/kg K
- 0.004 mmol/kg Mn.

### Example 6 - Biological activity

The preventive and curative neuroprotective properties of the pluri-ionic compositions defined below have been studied on in vitro cultures of sensory neurons.

Sensory neurons are part of the peripheral nervous system. Their cell bodies are located in the Dorsal Spinal Ganglions ("Dorsal Rooth Ganglia" D.R.G.) along the spinal cord and will emit extensions to the most distal extremities. One of the properties of sensory neurons is their ability to regenerate their extensions after section of nerves. This property is related to the presence of Schwann cells, nourishing and myelinating cells of the peripheral nervous system that release specific growth factors for the growth of axons.

The in vitro model used is a culture of sensory neurons myelinated by Schwann cells (Callizot et al.,2011, Exp Cell Res 317:2374-2383). This model, miniaturized in 96 wells, makes it possible to analyze the effects of molecules on the development of sensory neurons and on the myelination of axons by Schwann cells.

Protocol of the coculture of sensory neurons and Schwann cells: Female rats with 15 days gestation were killed by cervical dislocation (Wistar rats; January Lab) and the fetuses were removed from the uterus. Embryonic DRGs were collected and placed in an icy medium of Leibovitz 15 (L15; PanBiotech, Ref P04-27055, batch: 4590720) containing 2% Penicillin-Streptomycin (PS; PanBiotech, ref: P06-07100, batch: 7390220) and 1% bovine serum albumin (BSA; PanBiotech, Ref: P06-1391100, batch: H200403). DRGs were dissociated by trypsinization for 20 minutes (min) at 37°C (Trypsin EDTA 1X; PanBiotech, Ref: P10-023100, batch: 3590720). The reaction was stopped by the addition of Dulbecco's modified Eagle medium (DMEM; PanBiotech, Ref: P04-03600, batch: 4080720) containing DNase I grade II (0.1 mg/ml; PanBiotech, Ref: P60-37780100, batch: H181015) and 10% fetal calf serum (FCS; Invitrogen, Ref: 10270106, batch: 2232584). The cells were then mechanically dissociated by 3 passes through a 10 ml pipette. The cells were then centrifuged at 180 x g for 10 min at + 4 ° C on a layer of BSA (3.5%) in medium L15. The supernatant was discarded and the cell pellets were resuspended in a defined culture medium consisting of Neurobasal (Invitrogen, Ref: 21103049, batch: 2176355) supplemented with 2% B27; (Invitrogen, ref: 17504-044, batch: 2193553), L-glutamine (2 mM; PanBiotech, Ref: P04-80100, batch: 3301019), 2% PS and 50ng/mL NGF (Sigma, Ref: N1408, L batch batch: SLCG1641). The viable cells were then counted in a Neubauer cytometer using the trypan blue exclusion test. The cells were seeded at a density of 12000 to 20000 cells/wells in 96-well plates (pre-coated with poly-D-lysine; Greiner, Ref: 655940, batch: E20093UL) and have been grown at 37°C in humidified air (95%) / CO2 (5%) atmosphere. Half of the middle was changed every 2 days with fresh middle. The cells are held for 7 to 12 days to allow the proliferation of Schwann cells and sensory neurons. On day 8, the medium is supplemented with 50 µg/mL of ascorbic acid (AA; Sigma, Ref 092902, batch: 05316HJ-438) to initiate the differentiation of Schwann basal cells into myelinating Schwann cells.

Under these conditions, after 5 days of culture with AA, the myelin sheaths are detected with an anti-MAG antibody (Myelin antigen glycoprotein), an early marker of myelin.
A. Study of the neuroprotective properties of pluri-ionic compositions on myelinated sensory neurons after Cisplatin poisoning: curative protocol

The aim of this study is to evaluate the neuroprotective effect of pluri-ionic compositions, at 3 different lithium concentrations, after cisplatin poisoning of myelinated sensory neurons in culture. In this culture, the pluri-ionic composition will be incubated at the same time as cisplatin.

### A.1. Protocol

### A.1.1. Preparation, exposure and treatment of cisplatin: curative protocol

Cis-diammineplatin (II) dichloride (cisplatin; Sigma, ref: P4394, batch: MKCL0026) was reconstituted in medium at 10 mg /ml (stock solution). The control environment was prepared under the same conditions. After 12 days of culture with AA, the primary sensory neurons were treated for 24 hours with pluri-ionic compositions in the presence or absence of cisplatin poisoning. The cisplatin preparation was used on neurons at a final concentration of 12 µM diluted in control medium for 24 hours of incubation. The pluri-ionic compositions tested are obtained by dissolution in water of lithium chloride, magnesium chloride hexahydrate and potassium chloride, and contain:
1. KY21400: Li 10.37 mmol/kg, Mg 2.27 mmol/kg, K 16.88 mmol/kg
2. KY21310: Li 5.76 mmol/kg, Mg 1.26 mmol/kg, K 9.38 mmol/kg
3. KY21200: Li 1.73 mmol/kg, Mg 0.38 mmol/kg, K 2.81 mmol/kg

The following conditions were tested:
- Control medium
- Control + cisplatin (12µM, 24h)
- Pluri-ionic compositions KY21400, KY21310 or KY21200 + cisplatin (12µM, 24h)
- Pluri-ionic compositions KY21400, KY21310 or KY21200
- Control + individual ions at various concentrations
- Control + cisplatin (12µM, 24h) + individual ions at various concentrations

The culture was carried out with 6 wells per condition.

A.1.2. Primary endpoint: measure of the total number of sensory neurons and assessment of neurite length and myelin sheath length on sensory neurons after cisplatin intoxication and without intoxication.

After 24 hours of treatment in the presence or absence of cisplatin, the cells were fixed by a solution of acetic acid / ethanol (5/95) for 5 min at -20 ° C, the control conditions were also fixed following the same procedure. The cells were then permeabilized and the non-specific sites were blocked with a saline phosphate buffer solution (PBS; PanBiotech; ref: P04-36500, batch: 2620121) containing 0.1% saponin (Sigma; ref: S7900, batch: BCBL8667V) and 1% FCS for 15 min to 30 min at room temperature. The cells were then incubated with a rabbit anti-neurofilament polyclonal antibody (NF; 1/500, Sigma; ref: N0142, batch: 083M4833) and with an anti-MAG mouse monoclonal antibody (1/400, Sigma; ref: MAB1567, batch: 3227322) in a PBS solution containing 1% FCS, 0.1% saponin, for 12 hours at 4°C.

For the evaluation of the toxicity of individual ions, the cells are fixed after 3 days of treatment. The cells are incubated with an anti-beta-tubulin mouse monoclonal antibody (1/2000, Sigma; Ref T8660-.2mL; batch: 034M4790V) in a blocking buffer at 4°C for 12 hours.

These antibodies were revealed with an Alexa Fluor 488 goat antimouse IgG (1/400, Molecular probe, ref: A11001, batch: 2247988) and an Alexa Fluor 568 goat rabbit anti-rabbit IgG (1/400, Molecular probe, ref: A11011, batch: 2017252) in PBS with 1% FCS, 0.1% saponin, for 1 hour at room temperature. The cell nuclei were labeled with a fluorescent marker (Hoechst's solution, Sigma; ref: H-33258, batch: 046M4048V) in the same buffer.

For each condition, 20 images per well were taken using InCell AnalyzerTM 2200 (GE Healthcare) with a magnification of 20x. The images of each well of culture were taken under the same conditions. The analysis was performed using Developer software (GE Healthcare). A total of 6 data per experimental condition was provided.

### A.1.3. Statistics

The data were expressed on average ± s.e. (out of 6 data per condition, 1 culture). A global analysis of the data was performed using a unidirectional analysis of variance (ANOVA) following the Dunnett's test.

The significance level is set at p <0.05. Results for which the p-value is greater than or equal to 0.05 are considered not significant ("n.s."), and therefore inconclusive.

### A.2. Results

### A.2.1. Effect of pluri-ionic composition on the survival of sensory neurons

Treatment with the pluri-ionic compositions alone, KY21400, KY21310 and KY21200 for 24 hours does not modulate cell survival (respectively 95%, 96% and 101% of the control).

Cisplatin at 12 µM induces a significant decrease in the survival of sensory neurons (61% cell death with p <0.001).

Treatment with the pluri-ionic compositions KY21400, KY21310 and KY21200, during the 24h of cisplatin lesion, is able to partially and significantly prevent cell death of neurons (respectively 32% with p <0.05, 31% with p <0.05 and 32% of dead cells with p <0.05).

### A.2.2. Effect of pluri-ionic compositions on the length of neurites of sensory neurons

Treatment with the pluri-ionic compositions KY21400, KY21310 and KY21200 for 24 hours on healthy cells, induces a slight but not significant increase in the length of the neurites on sensory neurons (respectively 31%, 34% and 54% of the control).

Cisplatin at 12 µM induces a significant decrease in the length of neurites on sensory neurons (65% loss of neurites with p <0.01).

Treatment with the pluri-ionic compositions according to the invention KY21310 and KY21200 is able to effectively preserve the length of neurites of sensory neurons of cisplatin lesions for 24 hours (respectively 19% with p <0.05 and 11% loss of neurites with p <0.05).

However, treatment with the pluri-ionic composition KY21400 is able to partially but not significantly preserve the length of neurites of the sensory neurons of the cisplatin lesion for 24 hours (28% of neurite loss).

In parallel, the effect of isolated or combined ions two by two on the survival of sensory neurons and the length of neurites was also evaluated under the same conditions. The solutions are all prepared from the chlorides of the ions tested.

The results are shown in Table 1.

**Table 1.**

| | | Mean percentage of sensory neurons number relative to control | Mean percentage of neurite length relative to control |
|---|---|---|---|
| 1 | Control + cisplatin (12µM, 24h) | 39% | 35% |
| 2 | KY21400 + cisplatin (12µM, 24h) | 68% (p<0.05) | 72% (n.s.) |
| 3 | KY21310 + cisplatin (12µM, 24h) | 69% (p<0.05) | 81% (p<0.05) |
| 4 | KY21200 + cisplatin (12µM, 24h) | 68% (p<0.05) | 89% (p<0.05) |
| 5 | KY21400 (Li 10.37 mmol/kg, Mg 2.27 mmol/kg, K 16.88 mmol/kg) | 95% (n.s.) | 131% (n.s.) |
| 6 | KY21310 (Li 5.76 mmol/kg, Mg 1.26 mmol/kg, K 9.38 mmol/kg) | 96% (n.s.) | 134% (n.s.) |
| 7 | KY21200 (Li 1.73 mmol/kg, Mg 0.38 mmol/kg, K 2.81 mmol/kg) | 101% (n.s.) | 154% (n.s.) |
| 8 | K 366 mg/kg= 9.38 mmol/kg | 117% (n.s.) | 115% (n.s.) |
| 9 | Li 40 mg/kg= 5.8 mmol/kg | 97% (n.s.) | 77% (p<0.0001) |
| 10 | Mg 30.8 mg/kg= 1.27 mmol/kg | 108% (n.s.) | 98% (n.s.) |
| 11 | Li 72 mg/kg= 10.4 mmol/kg + Mg 54.4 mg/kg= 2.24 mmol/kg | 29% (p<0.0001) | 24% (p<0.0001) |
| 12 | Li 40 mg/kg= 5.8 mmol/kg + Mg 30.8 mg/kg= 1.27 mmol/kg | 24% (p<0.0001) | 29% (p<0.0001) |
| 13 | Li 40 mg/kg= 5.8 mmol/kg+ K 336 mg/kg = 9.38 mmol/kg | 0% (p<0.0001) | 1% (p<0. 0001) |
| 14 | Li 40 mg/kg= 5.8 mmol/kg + Mg 54.4 mg/kg= 2.24 mmol/kg + K 660 mg/kg = 16.88 mmol/kg | 101% (n.s.) | 94% (n.s.) |

Lines 12 to 13 of the table show that lithium combined with only one of the magnesium or potassium ions is highly toxic at 40 mg/kg, whereas it is no longer toxic in the presence of the other two ions at the same concentrations. So there is really a synergistic effect of the combination of the three ions lithium, magnesium and potassium.

The ability of isolated ions to preserve the survival of sensory neurons and the length of neurites was tested in parallel. The results are shown in Table 2.

**Table 2.**

| | | Mean percentage of sensory neurons number relative to control | Average percentage of neurite length relative to control |
|---|---|---|---|
| 1 | Control + cisplatin (12µM, 24h) | 51-57% | 63-75% |
| 2 | Control + cisplatin (12µM, 24h) + K 366 mg/kg= 9.38 mmol/kg | 58% | 91% |
| 3 | Control + cisplatin (12µM, 24h) + Li 40 mg/kg= 5.8 mmol/kg | 66% | 80% |
| 4 | Control + cisplatin (12µM, 24h) + Mg 1.5 mg/kg | 71% | 67% |

None of the individual ions significantly preserve sensory neurons from cisplatin-induced apoptosis, whereas the pluri-ionic combinations tested at similar concentrations do. The same is true for combinations of potassium and magnesium, which do not significantly improve the survival of sensory neurons. This difference is probably due to the reduction in the toxicity of the pluri-ionic combinations compared to the isolated ions, which allows the protective effect to prevail over the toxicity.

Manganese was also tested under the same conditions with concentrations ranging from 0.15 mg/kg to 1.2 mg/kg, without demonstrating any significant effect on neuron survival or neurite length.

Other combinations of lithium-magnesium-potassium according to the invention have also been tested. They are shown in Table 3.

**Table 3.**

| | Magnesium | | | Lithium | | Potassium | | |
|---|---|---|---|---|---|---|---|---|
| | mg/kg | mmol/kg | Molar vs Li ratio | mg/kg | mmol/kg | mg/kg | mmol/kg | Molar vs Li ratio |
| C1 | 30.8 | 1, 27 | 0.22 | 40 | 5,76 | 300 | 7,7 | 1,33 |
| C2 | 30, 8 | 1, 27 | 0, 22 | 40 | 5,76 | 500 | 12,79 | 2,22 |
| C3 | 30, 8 | 1, 27 | 0, 22 | 40 | 5,76 | 366, 7 | 9,38 | 1, 63 |
| C4 | 30, 8 | 1, 27 | 0, 22 | 40 | 5,76 | 350 | 9 | 1,56 |
| C5 | 5, 97 | 0, 2 | 0, 14 | 12 | 1, 7 | 110 | 2, 8 | 1, 63 |
| C6 | 9, 2 | 0, 37 | 0, 24 | 12 | 1, 7 | 110 | 2, 8 | 1, 63 |
| C7 | 9, 2 | 0, 37 | 0, 24 | 12 | 1, 7 | 118, 65 | 3, 03 | 1,76 |
| C8 | 9, 2 | 0, 37 | 0, 24 | 12 | 1, 7 | 115,25 | 2,94 | 1,73 |
| C9 | 46 | 1, 9 | 0, 22 | 60 | 8, 6 | 593,19 | 1,52 | 1,76 |
| C10 | 62,73 | 2,56 | 0, 30 | 60 | 8, 6 | 511,89 | 13,09 | 2,12 |
| C11 | 67.21 | 2,76 | 0, 32 | 60 | 8, 6 | 550 | 14,07 | 1, 64 |
| C12 | 30,8 | 1, 3 | 0, 22 | 40 | 5,76 | 600 | 15, 3 | 2, 66 |
| C13 | 30,8 | 1, 3 | 0, 22 | 40 | 5,76 | 250 | 6, 4 | 1, 11 |
| C14 | 50 | 2, 10 | 0, 36 | 40 | 5,76 | 366, 7 | 9,38 | 1, 63 |
| C15 | 15 | 0, 6 | 0, 11 | 40 | 5,76 | 366, 7 | 9,38 | 1, 63 |
| C16 | 50 | 2, 1 | 0, 36 | 40 | 5,76 | 250 | 6, 4 | 1, 11 |

The compositions C12 to C16 are outside the ranges of molar ratios according to the invention, while the other compositions, C1 to C11, are representative of the algorithm of the invention.

The effect of ion combinations on sensory neuron survival and neurite length was evaluated under the same conditions as described above. The solutions are all prepared from the chlorides of the tested ions.

The results are shown in Table 4.

**Table 4.**

| | Mean percentage of sensory neurons number relative to control |
|---|---|
| Control + cisplatin (12mm, 24h) | 53. 1 % |
| Control + cisplatin (12mm, 24h) + C1 | 82.13%** (p<0.01) |
| Control + cisplatin (12mm, 24h) + C2 | 81.66%** (p<0.01) |
| Control + cisplatin (12mm, 24h) + C3 | 79.42%* (p< 0.05 ) |
| Control + cisplatin (12mm, 24h) + C4 | 78.47%* (p<0.05) |
| Control + cisplatin (12mm, 24h) + C5 | 68.71%* (p<0.05) |
| Control + cisplatin (12mm, 24h) + C6 | 65.05%* (p<0.05) |
| Control + cisplatin (12mm, 24h) + C7 | 69.51%** (p<0.01) |
| Control + cisplatin (12mm, 24h) + C8 | 66.06%* (p<0.05) |
| Control + cisplatin (12mm, 24h) + C9 | 90.21%** (p<0.01) |
| Control + cisplatin (12mm, 24h) + C10 | 84.18%** (p<0.01) |
| Control + cisplatin (12mm, 24h) + C11 | 80.04%* (p<0.05) |
| Control + cisplatin (12mm, 24h) + C12 | 69.53% (n.s.) |
| Control + cisplatin (12mm, 24h) + C13 | 69.13% (n.s.) |
| Control + cisplatin (12mm, 24h) + C14 | 70.77% (n.s.) |
| Control + cisplatin (12mm, 24h) + C15 | 67.69% (n.s.) |
| Control + cisplatin (12mm, 24h) + C16 | 62.19% (n.s.) |

The above results demonstrate that the compositions of the invention significantly reduce the apoptosis of sensory neurons induced by cisplatin over all the claimed ranges. When potassium and / or magnesium are below and / or above the ranges of molar ratios according to the invention (C12 to C16), the combination no longer works.

### A.2.3. Effect of pluri-ionic compositions on the length of the myelin sheath of sensory neurons (preventive protocol)

Treatment with the pluri-ionic compositions KY21400, KY21310 and KY21200 for 24 hours on healthy cells does not modulate the length of the myelin sheath of sensory neurons (respectively 89%, 97% and 89% of the control).

Cisplatin at 12 µM induces a significant decrease in the length of the myelin sheath of sensory neurons (78% of myelin loss with p <0.001).

Treatment with the pluri-ionic compositions KY21310 and KY21200 significantly preserves the length of the myelin sheath of the sensory neurons from cisplatin lesions for 24 hours (respectively 46% with p <0.05 and 34% loss of myelin with p <0.01).

However, treatment with KY21400 pluri-ionic composition induces a slight but non-significant increase in myelin sheath length after cisplatin injury for 24 hours (66% of myelin loss).

### B. Neuroprotective effect of pluri-ionic compositions on rat myelinated primary sensory neurons after cisplatin injury: protection protocol

The aim of this study was to investigate the neuroprotective effect of pluri-ionic compositions after cisplatin injury on rat myelinated sensory neurons.

### B.1. Protocol

### B.1.1. Preparation, exposure and treatment of cisplatin

Cis-diammineplatin (II) dichloride (cisplatin; Sigma, ref: P4394, batch: MKCL0026) was reconstituted in medium at 10 mg /ml (stock solution). The control environment was prepared under the same conditions. After 12 days of culture with AA, the primary sensory neurons were pretreated for 2 hours with the pluri-ionic composition at 3 concentrations and then intoxicated by cisplatin. The cisplatin preparation was used on neurons at a final concentration of 12 µM diluted in control medium for 24 hours of incubation.

The pluri-ionic compositions tested contained, in water:
1. KY21400: Li 10.37 mmol/kg, Mg 2.27 mmol/kg, K 16.88 mmol/kg
2. KY21310: Li 5.76 mmol/kg, Mg 1.26 mmol/kg, K 9.38 mmol/kg
3. KY21200: Li 1.73 mmol/kg, Mg 0.38 mmol/kg, K 2.81 mmol/kg

The following conditions were assessed:
- Control medium
- Control + cisplatin (12µM, 24h)
- Pluri-ionic compositions KY21400, KY21310 or KY21200 + cisplatin (12µM, 24h)
- Pluri-ionic compositions KY21400, KY21310 or KY21200.

This study was carried out with 6 wells per condition and duplicated for validation of the results. The data taken and analyzed are the added results of the 2 studies.

### B.1.2. Primary endpoint: measure of the total number of sensory neurons and assessment of neurite length and myelin sheath length on sensory neurons after cisplatin poisoning and without intoxication

After 24 hours of treatment in the presence or absence of cisplatin, the cells were fixed by a solution of acetic acid / ethanol (5/95) for 5 min at -20 ° C, the control conditions were also fixed following the same procedure. The cells were then permeabilized and the non-specific sites were blocked with a saline phosphate buffer solution (PBS; PanBiotech; ref: P04-36500, batch: 2620121) containing 0.1% saponin (Sigma; ref: S7900, batch: BCBL8667V) and 1% FCS for 15 min at room temperature. The cells were then incubated with a rabbit anti-neurofilament polyclonal antibody (NF; 1/500, Sigma; ref: N0142, batch: 083M4833) and with an anti-MAG mouse monoclonal antibody (1/400, Sigma; ref: MAB1567, batch: 3227322) in a PBS solution containing 1% FCS, 0.1% saponin, for 12 hours at 4°C.

These antibodies were revealed with an Alexa Fluor 488 goat antimouse IgG (1/400, Molecular probe, ref: A11001, batch: 2247988) and an Alexa Fluor 633 anti-goat rabbit IgG (1/400, Molecular probe, ref: A21070, batch: 1700326) in PBS with 1% FCS, 0.1% saponin, for 1 hour at room temperature. The cell nuclei were labeled with a fluorescent marker (Hoechst's solution, Sigma; ref: H-33258, batch: 046M4048V) in the same buffer.

For each condition, 20 images per well were taken using InCell AnalyzerTM 2200 (GE Healthcare) with a magnification of 20x. The images of each culture well were taken under the same conditions. The analysis was performed using Developer software (GE Healthcare). A total of 6 data per experimental condition was provided for each of the 2 studies.

### B.1.3. Statistics

The data were expressed on average ± e.g. (out of 6 data per condition per crop for 2 crops). A global analysis of the data was performed using a unidirectional analysis of variance (ANOVA) following the Dunnett's test. The significance level is set at p <0.05.

### B.2. Results

### B.2.1. Effect of pluri-ionic composition on the survival of sensory neurons

Treatment with the pluri-ionic compositions KY21400, KY21310 and KY21200 for 24 hours on healthy cells does not modulate cell survival (respectively 91%, 89% and 95% of the control).

Cisplatin at 12 µM induces a significant and significant decrease in neuron survival (64% of cell death with p <0.0001). Pretreatment with the pluri-ionic c omposition KY21400, KY21310 and KY21200, two hours before and during the 24 hours of cisplatin injury, makes it possible to partially and significantly save neurons from cell death (respectively 35% with p <0.0001, 30% with p <0.0001 and 30% of cell death with p <0.0001).

### B.2.2. Effect of pluri-ionic composition on the length of neurites of sensory neurons

Treatment with KY21400 pluri-ionic composition for 24 hours has no impact on the length of neurites of sensory neurons (111% of control).

Treatment with KY21200 pluri-ionic composition for 24 hours significantly increases the neurite length of sensory neurons (140% of control with p <0.01).

Cisplatin at 12 µM induces a significant decrease in the axonal length of sensory neurons (51% loss of neurites with p <0.0001).

A two-hour pretreatment with the pluri-ionic compositions KY21400, KY21310 and KY21200 is able to effectively preserve the neurite length of the sensory neurons of cisplatin lesion for 24 hours (respectively 83% with p <0.01, 100% with p <0.0001 and 102% of the control with p <0.0001).

### B.2.3. Effect of pluri-ionic composition on the myelin sheath length of sensory neurons

Treatment with the pluri-ionic compositions KY21400 and KY21200 for 24 hours on healthy cells induces a slight but not significant increase in the length of the myelin sheath of sensory neurons (respectively, 138% and 129% of control). Treatment with KY21310 pluri-ionic composition for 24 hours on healthy cells has no impact on the length of the myelin sheath (111% of the control).

Cisplatin at 12 µM induces a significant decrease in the length of the myelin sheath of sensory neuron extensions (69% of myelin loss with p <0.0001).

A two-hour pre-treatment with the pluri-ionic compositions KY21400, KY21310 and KY21200 significantly preserves the length of the myelin sheath of sensory neurons from cisplatin lesion during 24 hours (respectively 29% with p <0.001, 37% with p <0.01 and 29% loss of myelin with p <0.001).

In comparison, none of the individual ions at the concentrations used in the combinations KY21400, KY21310 and KY21200 demonstrated a significant protective effect on the myelin sheath length.

Manganese was also tested under the same conditions at concentrations ranging from 0.15 mg/kg to 1.2 mg/kg, without demonstrating any significant effect on the myelin sheath length.

### C. Analysis of the benefits of KY21400 pluri-ionic composition treatment of human keratinocytes cultured under Th2-stimulated conditions on a 2D sensitive skin model by transcriptomic screening of 93 genes

Objective: to assess the protective and restorative effects of pluri-ionic composition KY21400 (Li 10.37 mmol/kg, Mg 2.27 mmol/kg, K 16.88 mmol/kg) on an in vitro 2D keratinocyte model subjected to a cytokine cocktail mimicking a Th2-type environment using transcriptomic analysis (TLDA; TaqMan Low Density Array) of the regulation of gene expression of 93 epidermal targets (by RT-qPCR). This model, adapted from a 3D model described in the research article by Hubaux et al. 2018, makes it possible to reproduce in vivo disturbances as found in cutaneous atopic dermatitis and sensitive skin with an atopic tendency. This includes mild hyperproliferation and modification of a panel of genes related to inflammation, lipid homeostasis, skin barrier and itching.

The in vitro model uses cultures of epidermal keratinocytes derived from the normal human foreskin (NHEKs, Lonza, 00192906). The cells were cultured in Epilife medium (Fisher Scientific, M-EPI-500-A) supplemented with a human keratinocyte growth supplement (HKGS, Fisher Scientific, S-001-5) and antibiotics (Gentamycin, Fisher Scientific, 15710-049). The cells were kept in a humid incubator at 37°C with an atmosphere of 5% CO2.

### C.1.Protocol

### C.1.1. Inflamed model induction with Th2

The cells were stimulated with three Th2-bound cytokines (IL-4, IL-13 and IL-25), known to play an important role in the development of atopic dermatitis and to impair the function of the epidermal barrier. The three interleukins were applied to a sub-confluent cell monolayer at concentrations of 50 ng/ml for IL-4 and IL-13, and 20 ng/ml for IL-25, for 48 hours to induce gene expression modulations reminiscent of atopic dermatitis and sensitive skin.

C.1.2. Treatment with pluri-ionic composition KY21400 Simultaneously with stimulation with Th2, the cells were incubated with the KY21400 pluri-ionic composition, diluted in culture medium, then filtered and applied for 48 h.

### C.2. Analysis of changes in gene expression

### C.2.1. Total RNA extraction

At the end of the 48-hour treatment with Th2 stimulation and KY21400, total RNA was extracted using the Qiagen RNeasy kit (Qiagen; 74106). Cells were rinsed with cold PBS and lysed with the buffer provided in the kit. The extraction was carried out according to the manufacturer's instructions. The collected RNA was stored at -80°C.

### C.2.2. RNA integrity analysis

RNA concentration was determined by spectrophotometric measurement (QIAxpert, Qiagen) and RNA quality was analyzed by capillary electrophoresis (Agilent Bioanalyzer 2100 - Agilent RNA 6000 Nano Kit, 5067-1511).

The integrity of total RNA was assessed by visualizing intact ribosomal RNA bands. For the total RNA of the upper eukaryotes, the size of the ribosomal bands should be 1.9 kb for 18S RNA and 4.7 kb for 28S RNA. The intensity of the band corresponding to 28S RNA must be greater than the intensity of the band corresponding to 18S RNA. Small diffuse bands representing low molecular weight RNA (tRNA and 5S ribosomal RNA) may be present. RNA degradation will be apparent in the form of smearing of ribosomal RNA bands and high molecular weight background noise.

### C.2.3. cDNA synthesis

Reverse transcription was performed with the high-capacity RNA-to-cDNA kit (Applied Biosystems; 438706) from total RNA according to the manufacturer's instructions. The cDNA was then stored at -20°C until used in polymerase chain reactions.

### C.2.4. Validation of the test system by individual TaqMan assays

In order to control the impact of Th2 stimulation and validate the testing system, an amplification of selected targets that are reported in the literature to be regulated in response to that specific challenge or to be regulated in atopic dermatitis was performed. The 5 target genes are: ABCA1(ATP-binding cassette A1), CA2 (Carbonic anhydrase 2), CCL2(C-C chemokine ligand motif 2), NELL2 (Neural EGFL like 2) and POSTN (Periostin).

The target sequences of the genes of interest were amplified using TaqMan (Applied Biosystems) gene expression tests. These kits contain a TaqMan probe and two specific primers that have been pre-mixed at a concentration of 18 µM for each primer and 5 µM for the probe. This mixture is 20x concentrated. The TaqMan probes were grafted with a fluorophore (FAM) at their 5' end and with a 3' fluorescence quencher.

The PCRs were performed with the Quantstudio7 (Applied Biosystems) real-time PCR system. In short, 4 µl of cDNA (4 ng) was mixed with 10 µl of TaqMan Fast Advanced Master Mix (Applied Biosystems; 4444557), 1 µl of TaqMan Gene Expression Assay and 5 µl of water without RNAase. The thermal cycles were programmed with a first denaturation step at 95 ° C for 20s. The amplification protocol was followed by 40 cycles (1 s at 95°C and 20 s at 60°C).

In order to normalize the results, a household gene, YWHAZ(Tyrosine 3-Monooxygenase) was used on the same cDNA samples. A cDNA-free control was performed in parallel as a negative amplification control. This made it possible to verify the absence of contaminants. Relative expression levels were calculated by the comparative method Ct (ΔΔCt).

### 2.5. Management of TaqMan matrices, qPCR and Ct analysis

TaqMan qPCR microfluidic chips (or TaqMan low-density chips; TLDA) were designed by StratiCell and manufactured on demand by Applied Biosystems. Among the genes represented, 3 internal control genes or household genes, and 93 genes of interest were studied.

TaqMan chips have been processed according to the manufacturer's instructions (Micro Fluidic Card Getting Started Guide, Applied Biosystems).

The cDNA was mixed with a specific buffer (TaqMan Fast Advanced Master Mix, 4444557, Applied Biosystems) before being injected into the chips and dispersed into the wells by centrifugation. The chips were sealed and the qPCR was executed using the Quantstudio7 real-time PCR system (Applied Biosystems) and its software (QuantStudio Real-Time PCR v1.3. software, Applied Biosystems).

Threshold cycles (Ct) were obtained for each gene. The result files were exported from the qPCR device and analyzed using DataAssist software (v3.01, Applied Biosystems) designed to perform relative quantification of gene expression using the Ct (ΔΔCt) comparative method, via a combination of statistical analyses.

The data obtained for the untreated state stimulated by Th2 were compared to the untreated, unstimulated state. The condition stimulated by Th2 and treated with the pluri-ionic composition KY21400 was compared to the condition not treated/stimulated by Th2. Ct values were normalized to the average of the Cts of two household genes present on the chip (YWHAZ and B2M). The maximum threshold value for Ct was set at 36 cycles, which means that genes with Ct values greater than 36 were not considered in the analysis.

### C.2.6. Statistical analysis

For the statistical results, the analysis was performed by a paired Student's t-test for the comparison of the treated conditions with respect to the specific control condition with *p<0.05; **p<0.01 and ***p<0.001.

### C.3. Results

### C.3.1. Effect of the KY21400 pluri-ionic complex on gene markers of inflammation

Treatment with the KY21400 complex significantly counteracted the expression increases induced by Th2 stimulation for the TNFAIP6 (p=0.0006), CXCL10 (p=0.009) and CXCL11 (p=0.0271) genes involved in the inflammatory response.

### C.3.2. Effect of the KY21400 pluri-ionic complex on gene markers of lipid homeostasis of human skin

Treatment with KY21400 induces a global modification of lipid homeostasis by differential modulations in the expression of genes involved in the synthesis of ceramides, fatty acids / triacylglycerols and cholesterols, their metabolism and transport within keratinocytes. Treatment with the KY21400 complex significantly reversed the effects of Th2 stimulation on the expression of the ABCG1 gene (p=0.0211), a decrease in the GPAT3 gene (p=0.0188), an increase in the expressions of the SULT1E1 (p=0.0096), FA2H (p=0.0385), NR1H2 (p=0.0045) genes involved in lipid skin homeostasis.

### C.3.3. Effect of the KY21400 pluri-ionic complex on gene markers of terminal keratinocyte differentiation and hyaluronic acid metabolism

Treatment with KY21400 significantly restores the expression of two genes for terminal differentiation of keratinocytes and hyaluronic acid metabolism, suggesting a favorable impact on the barrier properties and spongiosis encountered in atopic dermatitis and eczema. HAS3 and CASP14, genes involved in epidermal hydration and terminal keratinocyte differentiation, showed significant downregulation (p=0.0422) and upregulation (p=0.0346) gene expression with KY21400 treatment, respectively. KY21400 induces unexpected "life-saving" downregulation of the expression of the two genes SPRR1A (p=0.0025) and IVL (p=0.0054), involved in the formation of the corneal envelope. KY21400 downregulated the gene encoding the hyaluronic acid receptor CD44 (p=0.0378).

### C.3.4. Effect of the KY21400 pluri-ionic complex on gene markers of hypersensitivity and itching

Treatment of keratinocytes with the KY21400 complex reversed the observed effect following stimulation with a Th2 cocktail of cytokines sign of a preventive effect of the potential development of hypersensitivity and itching. Among the genes studied, three of them see their expression decrease, or even return to the basal level with treatment: NGF (p= 0.0141), ANOS1 (p = 0.0294) and SEMA3A (p = 0.0034).

### C.3.5. Effect of the KY21400 pluri-ionic complex on specific gene markers of atopic dermatitis

Treatment with KY21400 downregulated CA2 expression (p=0.0064) suggesting a specific anti-atopic dermatitis effect of this complex.

### D. Preliminary evaluation of topical treatment in subjects with diabetic peripheral neuropathic pain

A gel-cream according to the invention has been prepared for use by diabetic patients with peripheral neuropathic pain. The cream gel is prepared according to the method of example 5 and comprises 75.70% of aqueous solution obtained from 72 g of solution 1, whose pH is adjusted between 7 and 7.5, 136 g of solution 2 and 100 g of solution 3.

The final concentrations of the minerals analyzed by ICP-MS in the gel-cream are:
- 12 ppm of Li or 1.31 mmol / kg of Li (molar ratio = 1)
- 9.4 ppm mg or 0. 38 mmol/kg Mg (molar ratio = 0.29)
- 107 ppm of K or 2.72 mmol /kg of K (molar ratio = 2.07)
- 11.7 ppm of Si, and
- 0.31 ppm Mn.

Selected patients have a score of at least 4 based on the standard DN4 questionnaire used for diagnosis of neuropathic pain, diagnosis validated by electromyogram. Selected patients applied the cream gel in addition to their usual drug treatment (duloxetin, pregabalin, gabapentin,...) without changing the doses of treatment during the study.

For a month, the patients applied the gel-cream on the lower limbs down to below the knee, and possibly between the toes, at the rate of 2 times a day: in the morning and in the evening, the very first application taking place in the evening.

Evaluations were conducted:
- Before the first application
- 10 minutes after the first application
- Before the second application the next morning
- The 15^{th} day of use
- The 30^{th} day of use.

Evaluation consists of scoring on a scale of 0 (not present) to 10 (extremely present), six criteria representatives of the symptoms of diabetic peripheral neuropathic pain.

Patients include 13 women and 11 men with an average age of 63.8 years and have an average DN4 score of 6.6. 9 patients have had symptoms for 1 to 5 years, 9 patients have had symptoms for 5 to 10 years and 5 patients have had symptoms for more than 10 years. Results: After 1 month, 58% of patients (14) report a 30% or greater reduction in pain, of which 46% indicate a 50% or greater reduction in pain.

The details of the results at one month by type of painful symptom are given in Table 5 below.

**Table 5**

| Type of pain | heartburn | Electric shock | Tingling | Tingling (pine and needles) | itch |
|---|---|---|---|---|---|
| Number of patients with this symptom before treatment | 21 | 18 | 22 | 20 | 15 |
| patients indicating a reduction in symptoms of at least 30% | 57% | 50% | 64% | 65% | 60% |
| patients indicating a reduction in symptoms of at least 50% | 52% | 50% | 50% | 55% | 40% |

This study shows the effectiveness of the cream gel in reducing diabetic peripheral neuropathic pain beyond the simple placebo effect (estimated at 20%). No side effects were observed.

In addition, after one month, 16 patients spontaneously reported an improvement in their sleep quality and mood, an improvement in their standing balance and walking perimeter, as well as a recovery of plantar and leg sensitivity.

## Claims

1. Pluri-ionic composition comprising mineral salts, including at least one mineral salt of lithium, of magnesium and of potassium, **characterized by** the following molar ratios:
lithium 1 - magnesium [0.13 - 0.34] - potassium [1.20-2.40].

2. Pluri-ionic composition according to claim 1, wherein the lithium concentration is comprised between 0.1 and 10 mmol / kg.

3. Pluri-ionic composition according to claim 1 or 2, wherein the molar ratio of potassium is comprised between 1.55 and 1.75.

4. Pluri-ionic composition according to one of claims 1 to 3, wherein the molar ratio of magnesium is comprised between 0.15 and 0.30.

5. A composition according to one of claims 1 to 4, comprising lithium in the form of lithium chloride, lithium carbonate and / or lithium hydroxide.

6. A composition according to one of claims 1 to 5, comprising magnesium in the form of magnesium chloride, magnesium carbonate, magnesium hydroxide, magnesium oxide, magnesium sulfate and / or magnesium silicate.

7. A composition according to one of claims 1 to 6, comprising potassium in the form of potassium chloride, potassium bromide, potassium iodide, potassium phosphate, potassium carbonate, potassium hydroxide, potassium silicate and / or potassium sulfate.

8. Method of preparing the composition according to one of claims 1 to 7, comprising the steps of:
- If necessary, the pH of an aqueous solution is adjusted between 6.0 and 7.5;
- One or more concentrated solutions comprising at least one mineral salt of lithium, magnesium and potassium is added to the aqueous solution to obtain the following molar ratios:
lithium 1 - magnesium [0.13 - 0.34] - potassium [1.20-2.40].

9. Method according to claim 8, according to which is added one or more concentrated solutions comprising at least one mineral salt of lithium, magnesium and potassium at a temperature greater than 50 ° C.

10. Method according to one of claims 8 to 9, according to which, the concentrated solution comprising at least one mineral salt of lithium, magnesium and / or potassium is concentrated 100 to 1000 times relative to the final concentration of the composition.

11. Composition according to one of claims 1 to 7 for use as a medicament for human use.

12. Composition according to one of claims 1 to 7 for use as a veterinary drug.

13. Composition according to one of claims 1 to 7 for use as a medical device.

14. Composition for its use according to one of claims 11 to 13 topically.

15. Composition for its use according to one of claims 11 to 13 for the topical prevention and/or treatment of neurogenic inflammation.

16. Composition for its use according to claim 15, according to which neurogenic inflammation is associated with neuropathic pain, musculoskeletal pain, orofacial pain, post-herpetic pain, rosacea, psoriasis, atopic dermatitis, prurigo noduralis, urticaria, painful scars or not, diabetic foot and / or wounds.

## Patentansprüche

1. Pluriionische Zusammensetzung, umfassend Mineralsalze, einschließlich mindestens eines Mineralsalzes des Lithiums, des Magnesiums und des Kaliums, **gekennzeichnet durch** die folgenden molaren Verhältnisse:
Lithium 1 - Magnesium [0,13 - 0,34] - Kalium [1,20-2,40].

2. Pluri-ionische Zusammensetzung nach Anspruch 1, wobei die Lithiumkonzentration zwischen 0,1 und 10 mmol/kg liegt.

3. Pluriionische Zusammensetzung nach Anspruch 1 oder 2, wobei das molare Verhältnis von Kalium zwischen 1,55 und 1,75 liegt.

4. Pluriionische Zusammensetzung nach einem der Ansprüche 1 bis 3, wobei das molare Verhältnis von Magnesium zwischen 0,15 und 0,30 liegt.

5. Zusammensetzung nach einem der Ansprüche 1 bis 4, umfassend Lithium in Form von Lithiumchlorid, Lithiumcarbonat und/oder Lithiumhydroxid.

6. Zusammensetzung nach einem der Ansprüche 1 bis 5, umfassend Magnesium in Form von Magnesiumchlorid, Magnesiumcarbonat, Magnesiumhydroxid, Magnesiumoxid, Magnesiumsulfat und/oder Magnesiumsilikat.

7. Zusammensetzung nach einem der Ansprüche 1 bis 6, umfassend Kalium in Form von Kaliumchlorid, Kaliumbromid, Kaliumiodid, Kaliumphosphat, Kaliumcarbonat, Kaliumhydroxid, Kaliumsilikat und/oder Kaliumsulfat.

8. Verfahren zur Herstellung der Zusammensetzung nach einem der Ansprüche 1 bis 7, umfassend die Schritte:
- Falls erforderlich, wird der pH-Wert einer wässrigen Lösung zwischen 6,0 und 7,5 eingestellt;
- Eine oder mehrere konzentrierte Lösungen, die mindestens ein Mineralsalz aus Lithium, Magnesium und Kalium enthalten, werden der wässrigen Lösung zugesetzt, um die folgenden molaren Verhältnisse zu erhalten:
Lithium 1 - Magnesium [0,13 - 0,34] - Kalium [1,20-2,40].

9. Verfahren nach Anspruch 8, wonach eine oder mehrere konzentrierte Lösungen, die mindestens ein Mineralsalz aus Lithium, Magnesium und Kalium enthalten, bei einer Temperatur von mehr als 50 °C zugegeben werden.

10. Verfahren nach einem der Ansprüche 8 bis 9, wonach die konzentrierte Lösung, die mindestens ein Mineralsalz von Lithium, Magnesium und/oder Kalium umfasst, 100- bis 1000-mal relativ zur Endkonzentration der Zusammensetzung konzentriert ist.

11. Zusammensetzung nach einem der Ansprüche 1 bis 7 zur Verwendung als Arzneimittel für den menschlichen Gebrauch.

12. Zusammensetzung nach einem der Ansprüche 1 bis 7 zur Verwendung als Tierarzneimittel.

13. Zusammensetzung nach einem der Ansprüche 1 bis 7 zur Verwendung als Medizinprodukt.

14. Zusammensetzung für seine Verwendung gemäß einem der Ansprüche 11 bis 13 topisch.

15. Zusammensetzung zu seiner Verwendung nach einem der Ansprüche 11 bis 13 zur topischen Vorbeugung und/oder Behandlung neurogener Entzündungen.

16. Zusammensetzung für seine Verwendung nach Anspruch 15, wonach neurogene Entzündungen mit neuropathischen Schmerzen, Muskel-Skelett-Schmerzen, orofazialen Schmerzen, postherpetischen Schmerzen, Rosacea, Psoriasis, atopischer Dermatitis, Prurigo noduralis, Urtikaria, schmerzhaften Narben oder nicht, diabetischem Fuß und/oder Wunden verbunden sind.

## Revendications

1. Composition pluri-ionique comprenant des sels minéraux, dont au moins un sel minéral de lithium, de magnésium et de potassium, **caractérisée par** les rapports molaires suivants :
Lithium 1 - Magnésium [0,13 - 0,34] - Potassium [1,20-2,40].

2. Composition pluri-ionique selon la revendication 1, dans laquelle la concentration en lithium est comprise entre 0,1 et 10 mmol/kg.

3. Composition pluri-ionique selon la revendication 1 ou 2, dans laquelle le rapport molaire du potassium est compris entre 1,55 et 1,75.

4. Composition pluri-ionique selon l'une des revendications 1 à 3, dans laquelle le rapport molaire du magnésium est compris entre 0,15 et 0,30.

5. Composition selon l'une des revendications 1 à 4, comprenant du lithium sous forme de chlorure de lithium, de carbonate de lithium et/ou d'hydroxyde de lithium.

6. Composition selon l'une des revendications 1 à 5, comprenant du magnésium sous forme de chlorure de magnésium, de carbonate de magnésium, d'hydroxyde de magnésium, d'oxyde de magnésium, de sulfate de magnésium et/ou de silicate de magnésium.

7. Composition selon l'une des revendications 1 à 6, comprenant du potassium sous forme de chlorure de potassium, de bromure de potassium, d'iodure de potassium, de phosphate de potassium, de carbonate de potassium, d'hydroxyde de potassium, de silicate de potassium et/ou de sulfate de potassium.

8. Procédé de préparation de la composition selon l'une des revendications 1 à 7, comprenant les étapes suivantes :
- Si nécessaire, le pH d'une solution aqueuse est ajusté entre 6,0 et 7,5 ;
- Une ou plusieurs solutions concentrées comprenant au moins un sel minéral de lithium, de magnésium et de potassium sont ajoutées à la solution aqueuse pour obtenir les rapports molaires suivants :
Lithium 1 - Magnésium [0,13 - 0,34] - Potassium [1,20-2,40].

9. Procédé selon la revendication 8, selon lequel est ajoutée une ou plusieurs solutions concentrées comprenant au moins un sel minéral de lithium, de magnésium et de potassium à une température supérieure à 50°C.

10. Procédé selon l'une des revendications 8 à 9, selon laquelle, la solution concentrée comprenant au moins un sel minéral de lithium, de magnésium et/ou de potassium est concentrée 100 à 1000 fois par rapport à la concentration finale de la composition.

11. Composition selon l'une des revendications 1 à 7 pour une utilisation en tant que médicament à usage humain.

12. Composition selon l'une des revendications 1 à 7 pour une utilisation en tant que médicament à usage vétérinaire.

13. Composition selon l'une des revendications 1 à 7 pour une utilisation en tant que dispositif médical.

14. Composition pour son utilisation selon l'une des revendications 11 à 13 par voie topique.

15. Composition pour son utilisation selon l'une des revendications 11 à 13 pour la prévention topique et/ou le traitement de l'inflammation neurogène.

16. Composition pour son utilisation selon la revendication 15, selon laquelle l'inflammation neurogène est associée à des douleurs neuropathiques, des douleurs musculo-squelettiques, des douleurs orofaciales, des douleurs post-zostériennes, de la rosacée, du psoriasis, de la dermatite atopique, du prurigo noduralis, de l'urticaire, des cicatrices douloureuses ou non, du pied diabétique et/ou des plaies.
